# EUROPEAN PATENT SPECIFICATION

(11) **EP 0 386 044 B1**
(45) Date of publication and mention of the grant of the patent: **08.01.1997**
(21) Application number: 88909274.8
(22) Date of filing: 27.10.1988
(51) Int. Cl.: A61K 38/03

(54) **HYPOGLYCAEMIC PEPTIDES**
HYPOGLYCEMISCHE PEPTIDE
PEPTIDES HYPOGLYCEMIQUES

(30) Priority: 02.11.1987 AU 5195/87
(43) Date of publication of application: 12.09.1990
(73) Proprietor: MONASH UNIVERSITY, Clayton Victoria 3168 (AU)
(72) Inventor: HEARN, Milton, Thomas, William, Balwyn, VIC 3103 (AU); NG, Frank, Man-Woon, Vermont South, VIC 3133 (AU); ROBSON, Victoria, Marie, Jane, Brighton, VIC 3186 (AU); O'DONOGHUE, Michael, Francis, Warrandyte, VIC 3113 (AU); RAE, Ian, David, Mount Waverley, VIC 3149 (AU)
(74) Representative: Grünecker, Kinkeldey, Stockmair & Schwanhäusser Anwaltssozietät
(86) International application number: AU8800421
(87) International publication number: WO8904323

(56) References cited:
- AU-A- 8 796 175
- US-A- 4 496 521
- Chemical Abstracts, Vol 98, no. 24, issued 1983, June 13
- Chemical Abstracts, Vol 98, no. 11, issued 1983 March 14
- BIOCHEMISTRY, vol. 7, no. 11, November 1968, pages 4069-4075,
- INT. J. PEPTIDE PROTEIN RES., vol. 18, no. 3, September 1981, pages 318-323,
- INT: J. PEPTIDE PROTEIN RES., vol. 27, no. 1, January 1986, pages 51-60,
- CHEMICAL ABSTRACTS, vol. 102, 1985, pages 661-663,
- CHEMICAL ABSTRACTS, vol. 92, 1980 page 722, abstract no 198744m,

## Description

This invention relates to novel compounds with hypoglycaemic activity and to processes for producing these compounds.

### Background of the Invention

The principal factor controlling blood glucose levels is the hormone insulin. Diabetes may result either from insufficient production of insulin or from resistance to the action of circulating insulin.

It has been suggested that a peptide fragment of human growth hormone (hGH), derived from enzymic digestion of pituitary, can counter the effects of natural inhibitors of insulin (Australian patent application no. 87961/75 by Choay S.A.). This α-peptide chain, having the sequence leu-ser-arg-leu-phe-asp-asn-ala, is comprised of amino acids all of which have the L configuration.

This peptide, equivalent to hGH 6-13, was stated to be able to counteract the inhibition of insulin by somantin, a degradation product of growth hormone, and was thought to potentiate the effects of insulin, to induce synthesis of insulin receptor sites on cells, and to sensitize β cells in the islets of Langerhans to glucose.

However, it has subsequently been found that the peptide disclosed does not have these activities. In fact, inconsistency in demonstrating the claimed biological activities, due to the absence of detailed knowledge of the precise conformational structure of the active substance, prevented further development and validation of the original work by Bornsten and co-workers, inventors in respect of AU 87961/75, and this application lapsed.

One object or the present invention is to provide novel compounds with hypoglycaemic activity.

We have now prepared a family of novel β-imide linked L-aspartyl-L-asparaginyl peptides as shown in claim 1.

Furthermore, the in vitro and in vivo properties of these β-imido-L-aspartyl-L-asparaginyl peptides as potent hypoglycaemic agents with functional capabilities of potentiating insulin action are demonstrated. In this application we describe (i) the synthesis of these peptides via solid and solution phase peptide synthetic protocols; (ii) the purification and structural characterization of these peptides; (iii) the in vitro evaluation of these peptides in various biological assay systems; (iv) the evaluation of these peptides in lowering blood glucose levels in vivo.

### Brief Description of the Figures

Figure 1 represents a stereochemical view of an amidated form of a peptide according to the invention;
Figure 2 represents far ultraviolet circular dichroic spectra for synthetic peptide Pep (6-13) β-aspartimido form I(―)and synthetic peptide Pep (6-13) α-form (-----). Spectra recorded in water at 21°C using 1 mg/ml solutions in 0.1 mm cells.

The solid (___) and the broken (---) lines correspond to the synthetic [Asu]¹¹ - hGH[6-13] and all the L-α-hGH[6-13] peptides, respectively. Circular dichroism spectroscopy is measured with units of [θ]_{R} x 10⁻³ deg cm² dmol⁻¹.
Figure 3 represents an infra-red spectrum for active synthetic peptide (6-13) β-aspartimido form I. The spectrum was measured between 2000-1500 cm⁻¹ after the peptide sample (ca. 5 mg) was pressed into a KBr disc;
Figure 4 represents an infra-red spectrum for synthetic peptide Pep (6-13) α-form, inactive. The spectra were measured between 2000-1500 cm⁻¹. (a) the sample (ca. 5 mg) of peptide was pressed into a KBr disc. (b) the sample (ca. 5 mg) of peptide was saturated with Nujol to form a mull;
Figure 5 represents fast atom bombardment (FAB) mass spectrometric spectra of the synthetic peptides active β-aspartimido form I Pep (6-13) and inactive α-form Pep (6-13).

From the FAB-MS of the peptides, the m/e value of 917 corresponds to the [Asu]¹¹ - hGH[6-13] (imide) peptide, whilst the m/e value of 935 corresponds to the L-α-hGH[6-13] peptide.
Figure 6 represents ¹H N.M.R. spectrum of active β-aspartimido form I synthetic peptide Pep (6-13);

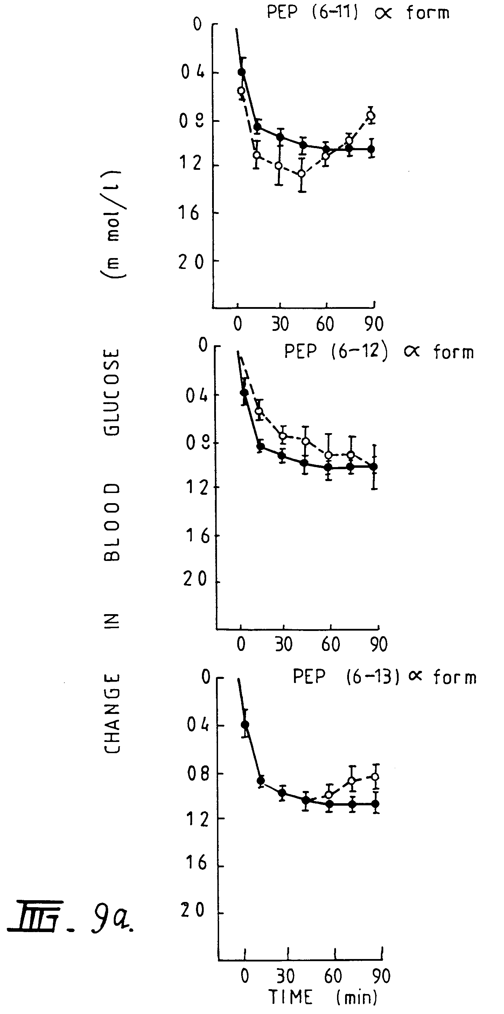

Figure 7 represents paper electrophoresis comparing synthetic peptide β-aspartimido form I Pep (6-13) active and synthetic peptide α-form dissolved in water and dilute (lM) NaOH.
   (a) Electrophoresis was performed at pH 6.5 for 1 h at 1.5 kV.
   (b) Electrophorosis was performed at pH 3.0 for 2.5 h at 1.5 kV.

Peptides were detected by spraying with ninhydrin;
Figure 8 represents HPLC chromatograms or synthetic Pep (6-13) peptides using a shallow gradient (0% - 20% Solvent B over 20 min).
   (a) Injection of purified synthetic Pep (6-13) β-aspartimido form I, active.
   (b) Injection of partly hydrolysed synthetic Pep (6-13) β-aspartimido form I.
   (c) Simultaneous injection of inactive synthetic Pep (6-13) α-form peptide and active β-aspartimido form I Pep (6-13) peptide α-form;
Figure 9 represents the change in blood glucose level in rats following injection of various synthetic inactive α form and active β-aspartimido forms of peptides according to the invention;
Figure 10 represents the effect of various doses of Pep (6-13) β imide during intravenous insulin sensitivity tests on normal rats;
Figure 11 represents the effect of Pep (6-13) on glucose tolerance tests in normal rats;
Figure 12 represents in vivo testing of hGh (6-13, Asn¹¹Asp¹²) (Figure 12a control peptide) and hGH (6-13, Asu¹¹Asp¹²) (Figure 12b test peptide) for insulin-potentiating activity on "untrained" female mice. The dotted lines represent control animals (injected with 0.2 ml of saline) in each case. The unbroken lines represent test animals (injected with 2.4 mg/Kg of peptide in 0.2 ml of saline). Insulin was injected at tₒ.
Figure 13 represents in vivo testing of peptides for insulin-potentiating activity on "untrained" and "trained" male mice. The dotted line represents the mean fall in blood glucose for control animals (injected with 0.2 ml of saline). The unbroken line represents the mean fall in blood glucose for test animals, injected with 2.3 mg/kg of peptide in 0.2 ml of saline, except for (a), in which the dose was 2.4 mg/kg.
   (a) Pep 6-13 (Gln¹¹Asp¹²), "untrained"
   (b) Pep 6-13 (Gln¹¹Asp¹²), "trained"
   (c) Pep 6-13 (Asn¹¹Asp¹²), "trained"
   (d) Pep 6-13 (Asp¹²), "trained" Insulin was injected at tₒ.

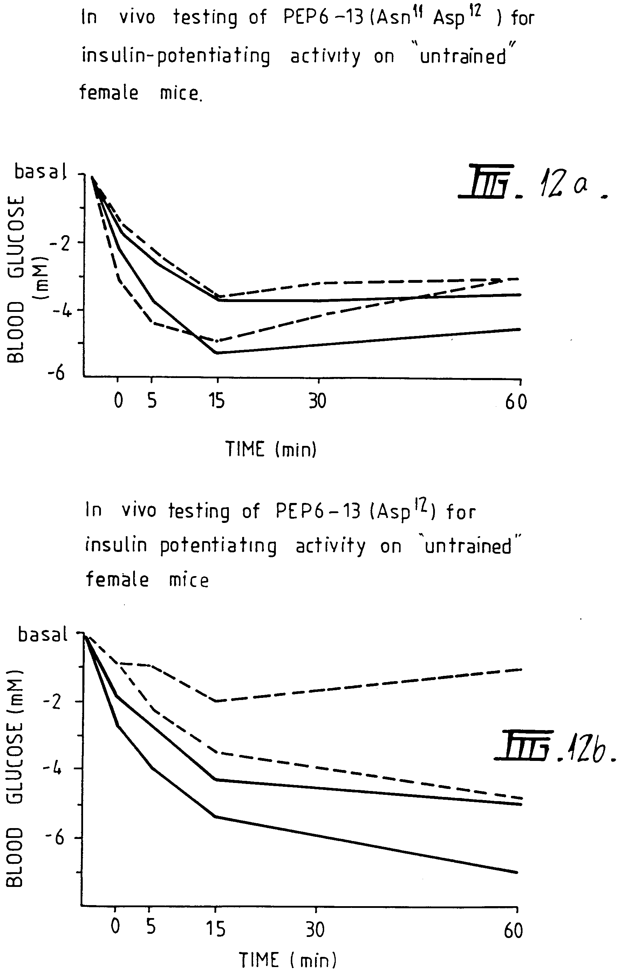

These data relate to the peptides with L-α-Gln L-α-Asn or L-α-Asp at position 11 in hGH[6-13]. These figures thus illustrate the, inactive control peptides with reference to the active hGH[6-13,Asu¹¹Asp¹²] (imide) anologue shown in Figure 12 (b).

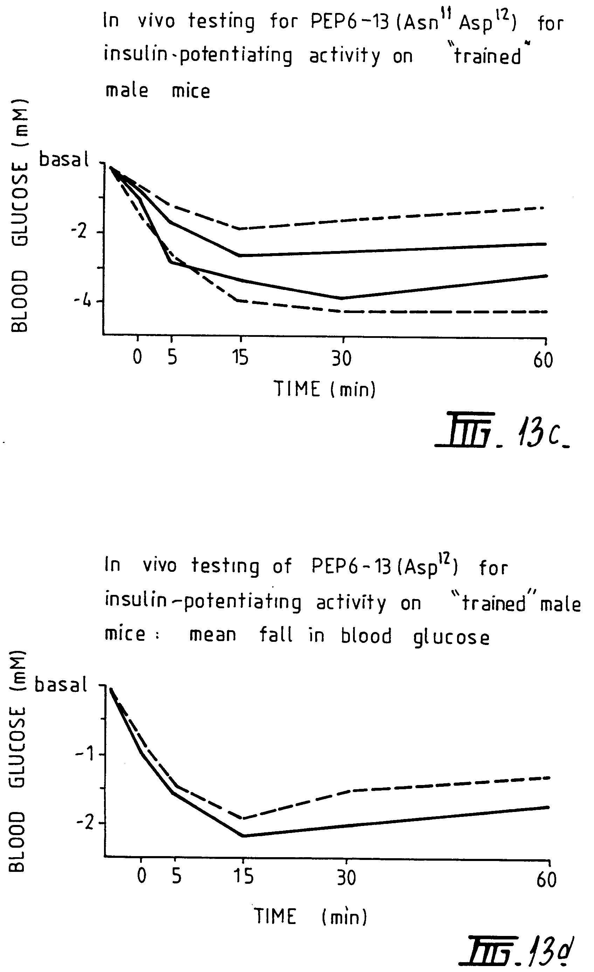

Figure 14 represents the in vitro effect of Pep (6-13) β-imide on glucose oxidation in isolated rat adipocytes in the absence and in the presence of various levels of insulin.

### Summary of the Invention

According to one aspect of the present invention there is provided a compound of general formula I; 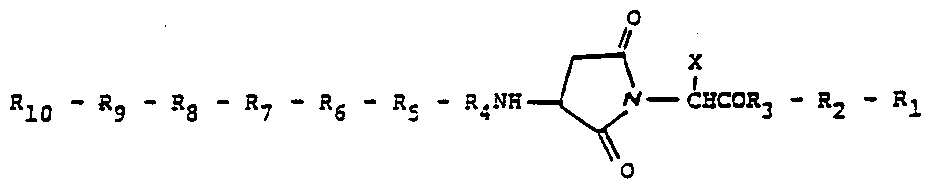 wherein X is hydrogen, -CH₂CONH₂ or -CH₂CH₂CONH₂;
each of R₁ and R₂ can be absent or is an L-α-amino acid, a δ-amino acid or an ε-amino acid;
R₃ is an L-α-amino acid, a δ-amino acid or an ε-amino acid;
R₄ is an L or D α-amino acid, a δ-amino acid or an ε-amino acid;
each of R₅ to R₇ is a bond, hydrogen or an L or D α-amino acid, a δ-amino acid or an ε-amino acid;
and each of R₈ to R₁₀ can be absent or is a hydrogen, or an L or D α-amino acid, a δ-amino acid or an ε-amino acid:
or a pharmaceutically acceptable salt thereof, with the proviso that the compound is not L-His-L-Ser-α-aminosuccinimido-Gly-L-Thr-L-Phe
or
L-Val-L-Tyr-L-Pro-α-aminosuccinimido-Gly-Ala.

Preferably R₅ is a L or D α-amino acid, a δ-amino acid or an ε-amino acid.

Preferably each of R₁, R₂ and R₃ is selected from the group consisting of alanine, glycine and phenylalanine.

Preferably R₄ is a hydrophobic amino acid; more preferably R₄ is selected from the group consisting of L-phenylalanine, L-tyrosine, L-tryptophan and L-histidine.

Preferably R₅ is a hydrophobic amino acid; more preferably R₅ is selected from the group consisting of L- or D- leucine, isoleucine and histidine.

R₆ is a basic amino acid, and is preferably selected from the group consisting of L- or D- arginine, lysine, and histidine.

R₇ is preferably a substantially hydrophobic amino acid, and is preferably selected from the group consisting of L- or D- serine, leucine and isoleucine.

R₈ is preferably selected from the group consisting of L- or D- leucine, phenylalanine, proline or isoleucine, an ε-amino acid such as 6-aminohexanoic acid, 4-aminocyclohexane-1-carboxylic acid or similar amino acids.

Each of R₉ and R₁₀ is preferably a substantially hydrophobic amino acid, and is most preferably selected from the group consisting of L- or D- leucine, phenylalanine, proline, isoleucine or tyrosine.

R₈, R₉ or R₁₀ may optionally be coupled to R₁ via a bifunctional agent such as 6-aminohexanoic acid. The amino acids may optionally have side chain substituents.

R₁, R₂, R₈, R₉, and or R₁₀ may be absent from the peptide, allowing coupling between C-terminal and N-terminal amino acids via a bifunctional agent such as 6-aminohexanoic acid.

R₆ is equivalent in terms of sequence alignment to residue 8 of human growth hormone.

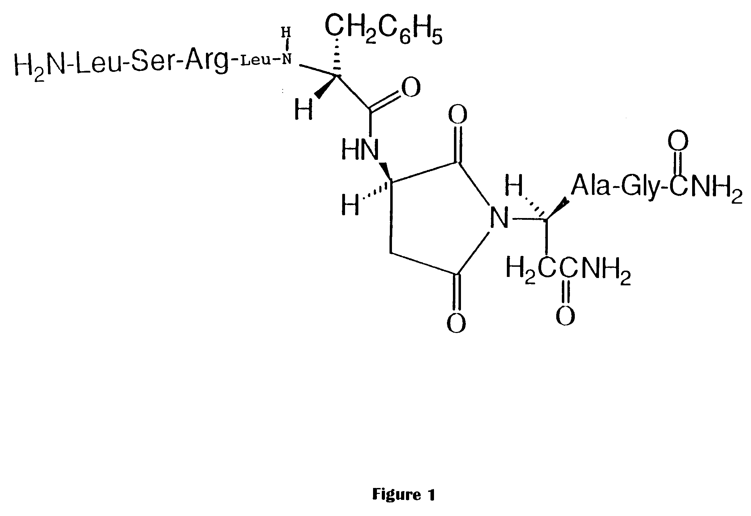

The cyclic imide structure type II β-turn as shown in general Formula I is essential for activity. Activity increases with chain length up to 8 residues. A stereochemical view of a representative peptide is shown in Figure 1.

The peptide according to the invention forms an amphipathic helix structure having a fluid hydrophobic face and a second face carrying an exposed imide group.

It will be apparent to persons skilled in the art that pharmacologically active analogues and derivatives of the compound of general formula I are within the scope of the invention, e.g. o-, m-, or p-aminobenzoic acid or α- or δ-lactams in place of the aspartimide ring.

According to a second aspect of the invention there is provided a method of solid phase synthesis of a peptide of general formula I comprising protection of the α-amino functionality of added amino acids using t-BOC or f-MOC and 5 treating the resin-bound protected α/straight chain L-aspartyl-L-asparaginyl peptide with a base to produce the stable β-imido form of the peptide.

The base is a dialkylamine such as piperidine, or a trialkylamine, preferably triethylamine.

The method of lowering the level of blood glucose in a mammal in need of such treatment comprises administering to that animal a hypoglycaemically effective dose of a compound of general formula I. Said compound may optionally be administered together with or in conjunction with a second hypoglycaemic agent.

### Detailed Description of the Invention

The invention will now be described in detail by way of example only, with reference to the accompanying drawings.

### 1. PEPTIDE SYNTHESIS

The various peptide analogues of the general formula I above were synthesised either by standard solution phase synthetic procedures or by using modifications of the Merrifield solid phase peptide synthesis procedure (R.B. Merrifield, J. Amer. Chem. Soc., 85, 2149-2145, 1963), of which the following protocols are representative. Peptides were initially synthesised in the alpha/straight chain form with all amino acid side chain groups protected. For the t-BOC-synthetic approach, the α-amino functionality of the incoming amino acid was protected with the tert-butyloxycarbonyl (t-BOC) group with deprotection, following coupling, with trifluoroacetic acid in CH₂Cl₂. Side chain protecting groups such as O-benzyl, tosylbenzylester and chlorobenzyloxycarbonyl were used for the trifunctional amino acids. The initial coupling to the benzhydrylamine resin employed a 3-fold excess of the designated side chain protected amino acid R₁, dissolved in N,N'-dimethylformamide (DMF). Subsequent couplings of the second, third, ..... eighth amino acid (counting asp and asn separately) employed a 2 fold excess of side chain protected amino acid in DMF. The amino acids were sequentially activated using tert-butanol and dicyclohexylcarbodiimide in approximately 5ml DMF and allowing reactants to stir at room temperature for 5-10 min. The acylurea was removed by filtration and the asymmetrical anhydride added to the resin. The suspended resin was stirred at room temperature for reaction times generally between 60 and 120 min. Each coupling was monitored for unreacted amino groups using 2,4,6-trinitrobenzenesulphonic acid. Similar synthetic strategies with the symmetrical protected amino acid anhydrides or the pentachlorophenylesters led to the preparation of identical products but in different yields. After the final coupling reaction was completed, the fully protected resin-bound peptide was treated with a reagent grade trialkylamine such as triethylamine (15- 20 ml per g resin) in order to convert the L-aspartyl-L-asparaginyl moiety to the stable β-imido form. The resin-bound peptide was side chain deprotected with trifluoroacetic acid, and the deprotected β-imido-L-aspartyl-L-asparaginyl peptide analogue cleaved from the resin using liquid hydrogen fluoride as the acid and dimethylsulphide/p-cresol or thioanisole as the scavenger. Reaction times were between 30 and 120 min at 0°C. Workup following cleavage involved standard procedures of peptide synthesis. The deprotected peptide was removed from the resin by washing with trifluoroacetic acid, then water, then water/acetonitrile mixtures.

The washes were then combined and lyophilised.

The crude dried product was chromatographed by anion exchange chromatography on Whatman DE 52 and then desalted on Biogel P2 gels. Further purification was achieved using reversed phase HPLC with octadecylsilica as stationary phase and gradients of 0-75% water-acetonitrile - 0.1% trifluoroacetic acid as mobile phase.

A similar synthetic strategy using f-MOC (9-fluorenylmethyloxycarbonyl group) protection has also been employed.

The synthetic peptides were characterised by amino acid compositional analysis, analytical reversed phase HPLC with several elution systems, fast atom bombardment mass spectroscopy (FAB-MS), paper electrophoresis at pH6.0, FTIR-infrared spectroscopy, aminopeptidase M cleavage, H¹-NMR spectroscopy and circular dichroism (CD) spectroscopy.

It was found that the synthesis protocol could be manipulated in order to produce all α or all imide forms of the peptide. In the absence of alkylamine treatment, all α was formed, and vice versa.

Representative results from these characterizations are illustrated for the peptide
leu-ser-arg-leu-phe-asp-asn-ala
(α-form, inactive), and for the peptide
leu-ser-arg-leu-phe-β-imidoasp-asn-ala
(β-imido form I, active).

Table 1 shows representative results of amino acid composition analysis following acid hydrolysis and aminopeptidase M cleavage.

**TABLE 1**

| Amino Acid Sequence | Leu | Ser | Arg | Leu | Phe | Asp | Asn | Ala |
|---|---|---|---|---|---|---|---|---|
| α-form Acid Hydrolysis^{a} | 1.03 | 0.78 | 0.93 | 1.03 | 0.98 | 0.98 | 0.98 | 0.96 |
| Enzymic Hydrolysis^{b} | 1.02 | 0.98 | - | 1.02 | 0.98 | 0.98 | 0.92 | 1.00 |
| aspartimide Acid Hydrolysis^{a} | 1.02 | 0.86 | 0.86 | 1.02 | 0.97 | 0.99 | 0.99 | 1.00 |
| Enzymic Hydrolysis^{b} | 1.06 | 0.99 | - | 1.06 | 1.00 | - | - | - |

| | | | | | | | | |
|---|---|---|---|---|---|---|---|---|
| a) 24 h acid hydrolysis at 110°C in 6M HCl. | | | | | | | | |
| b) 24 h aminopeptidase M digestion. | | | | | | | | |

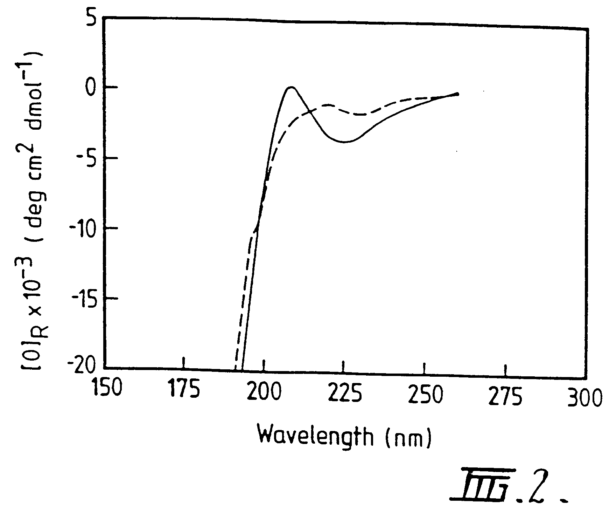

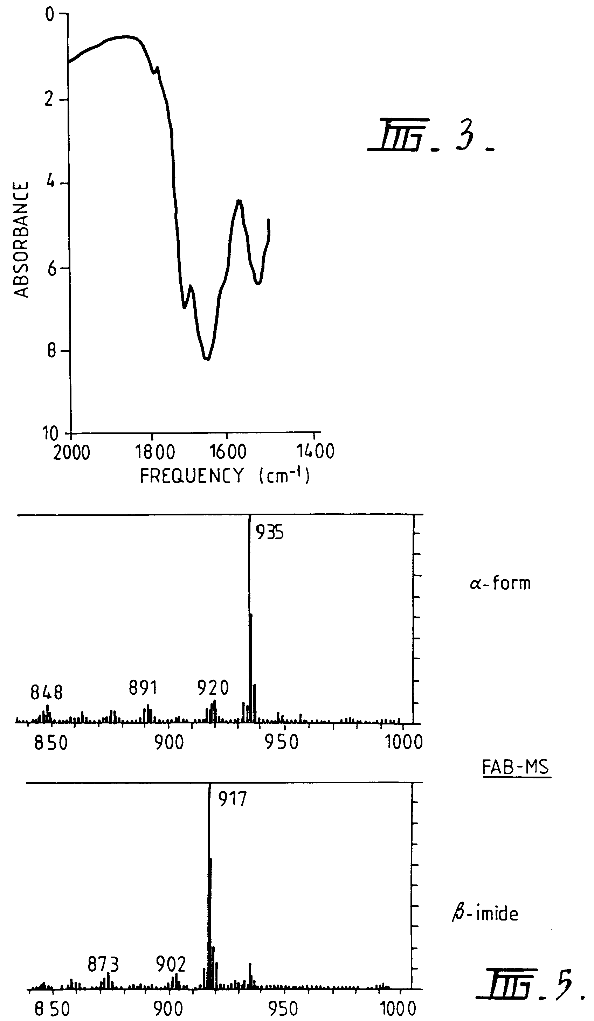

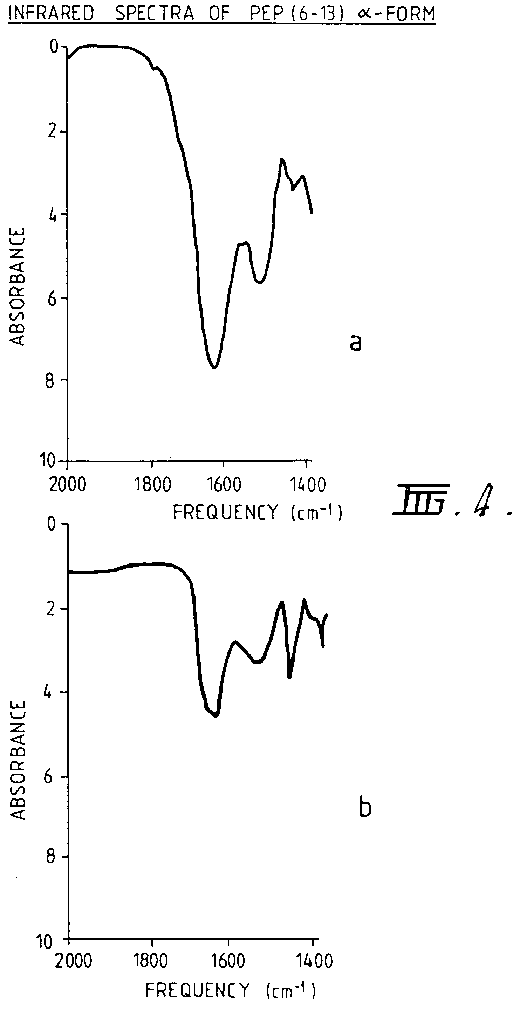

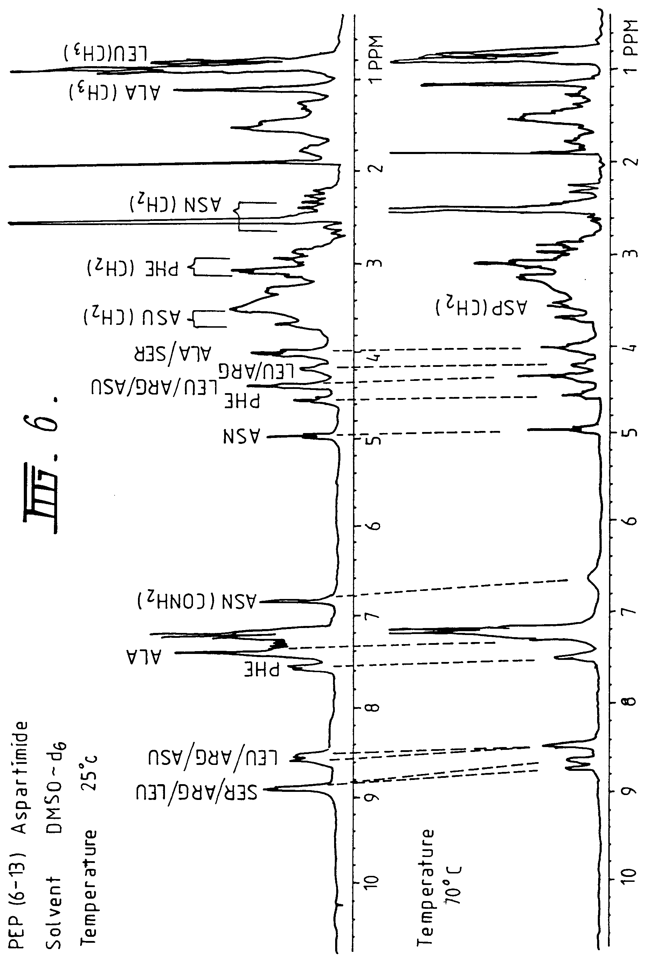

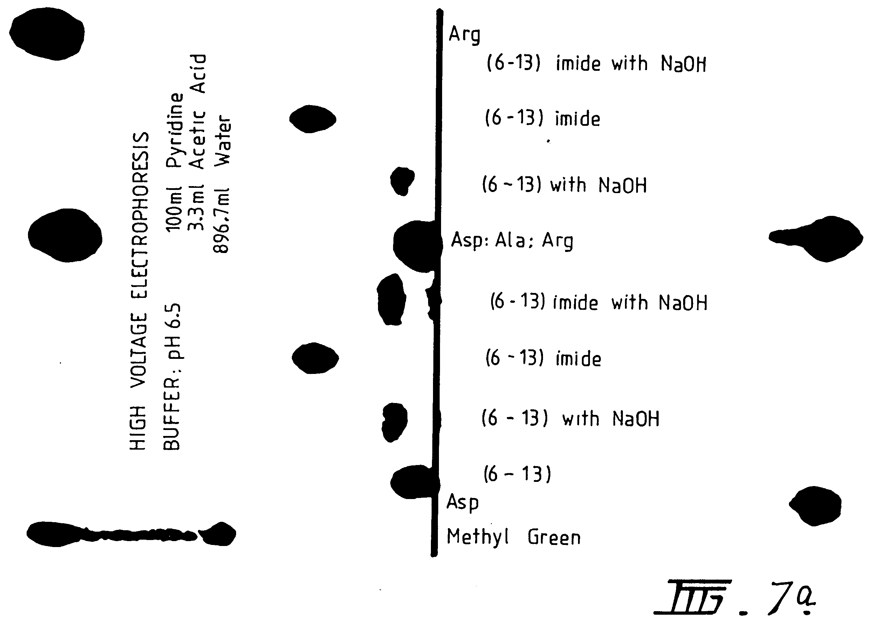

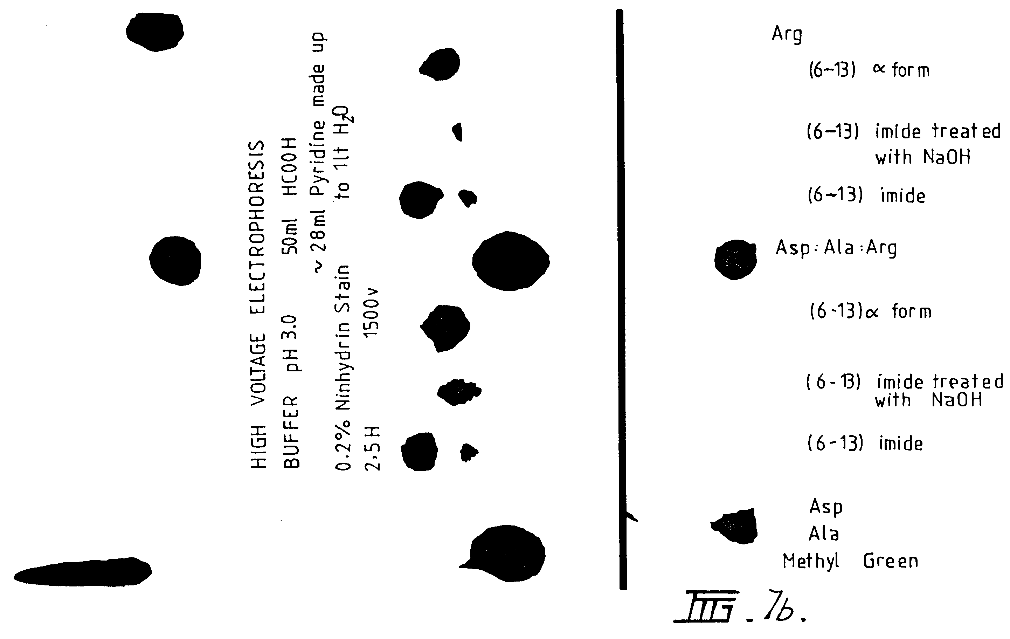

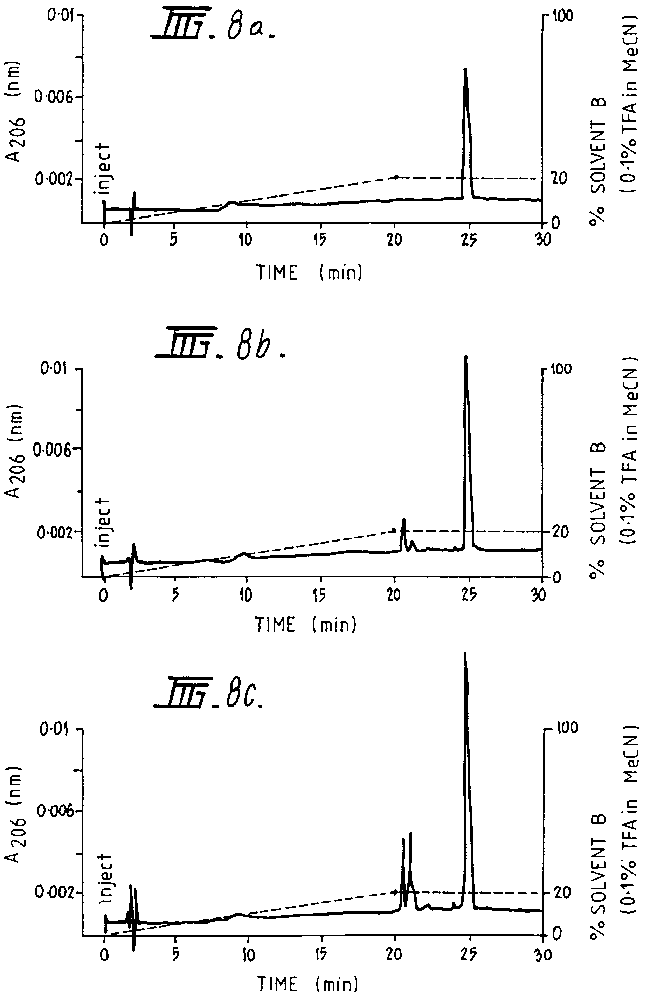

Results of circular dichroism spectroscopy, infrared spectroscopy, FAB-MS, and ¹H-NMR spectroscopy are illustrated in Figures 2 to 6 respectively. Without wishing to be bound by any proposed mechanism for the observed effects, it appears that the α form is in an extended configuration, while the β-aspartimido form I has one completely hydrophobic face, with the β-imido group at 90° to this hydrophobic face.

This conclusion is in accord with results obtained from correlation Overhauser spectroscopy (COSY) and nuclear Overhauser enhanced spectroscopy (NOESY) NMR spectroscopy for all cases of the representative active β-imide analogues.

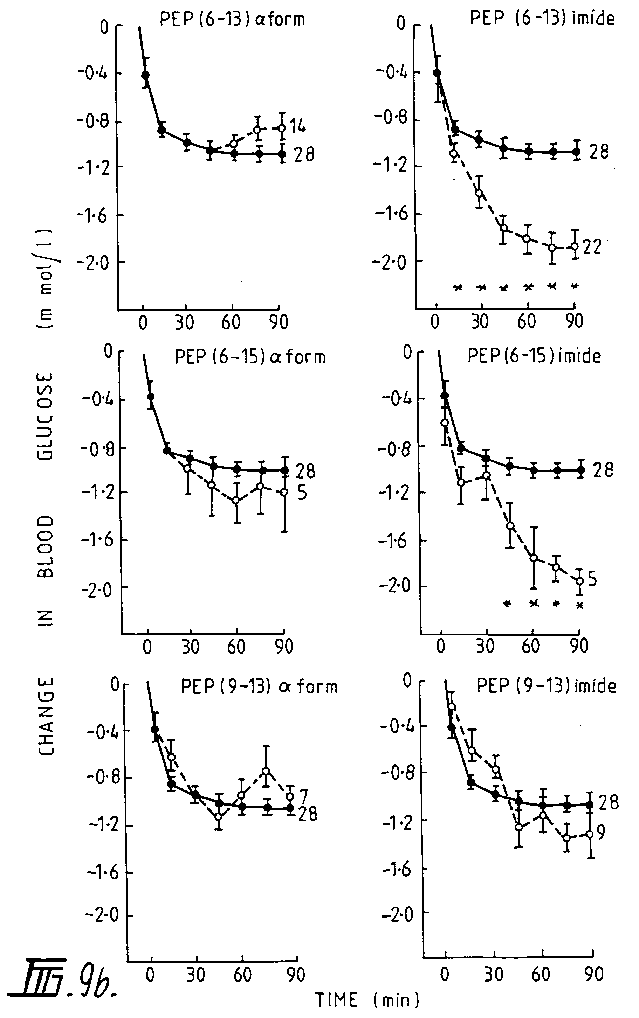

Figure 8 shows results of reversed phase HPLC on ODS-silica with shallow gradients of 0.1% trifluoracetic acid - acetonitrile-water.

### 2. BIOLOGICAL ACTIVITIES

Considering the physiology of hypoglycaemia and the possible molecular actions of peptide analogues with hypoglycaemic and insulin potentiating properties, the following in vitro and in vivo assays were chosen to assess the potency of the various synthetic β-imido-L-aspartyl-L-asparaginyl peptide analogues.

### (a) In vitro bioassays

(i) Activation of glycogen synthase
(ii) CO₂ production from U¹⁴C-glucose and ¹⁴C-pyruvate via pyruvate dehydrogenase
(iii) Glucose uptake

In these assays collagenase-dispersed rat adipocytes, cultured muscle cells (L6 rat skeletal muscle cell line) and hemidiaphragm preparations were employed.

### (b) In vivo bioassays

(i) Effect on basal blood glucose levels
(ii) Effect on insulin release
(iii) Intravenous insulin tolerance test
(iv) Activation of glycogen synthase

Representative data on the biological properties of the active β-imidoaspartylasparaginyl form I peptides and their inactive α-form peptide analogues are presented.

Table 2 shows the decrease in blood glucose level in rats following intravenous injection of peptides according to the invention. Injection of the α-form had no significant effect; however, injection of the β imide form of Pep (6-13) or Pep (6-15) resulted in a significant hypoglycaemic effect.

**TABLE 2**

| Peptide | Conc. of Peptide µmol/100g | No. of animals | Decrease in blood from basal | Difference from control mean | 2P Value t-test |
|---|---|---|---|---|---|
| Control | - | 28 | 1.05 | | |
| Pep (6-11) | 0.5 | 5 | 1.28 | 0.23 | N.S. |
| Pep (6-12) | 0.5 | 5 | 1.00 | -0.05 | N.S. |
| Pep (6-13) | 0.5 | 14 | 1.05 | 0.00 | N.S. |
| Pep (6-13) imide | 0.5 | 22 | 1.90 | 0.85 | < 0.001 |
| Pep (6-15) | 0.5 | 5 | 1.26 | 0.21 | N.S. |
| Pep (6-15) imide | 0.5 | 5 | 2.00 | 0.95 | < 0.001 |
| Pep (9-13) | 0.5 | 7 | 1.12 | 0.07 | N.S. |
| Pep (9-13) imide | 0.5 | 9 | 1.30 | 0.25 | N.S. |

Values are changes (means ± SEM) of blood glucose from the basal level (4.0 - 4.4 mmol glucose/l) at time 0. Differences between the control and test groups were statistically evaluated according to Student's t-test. N.S. indicates 2P > 0.05.

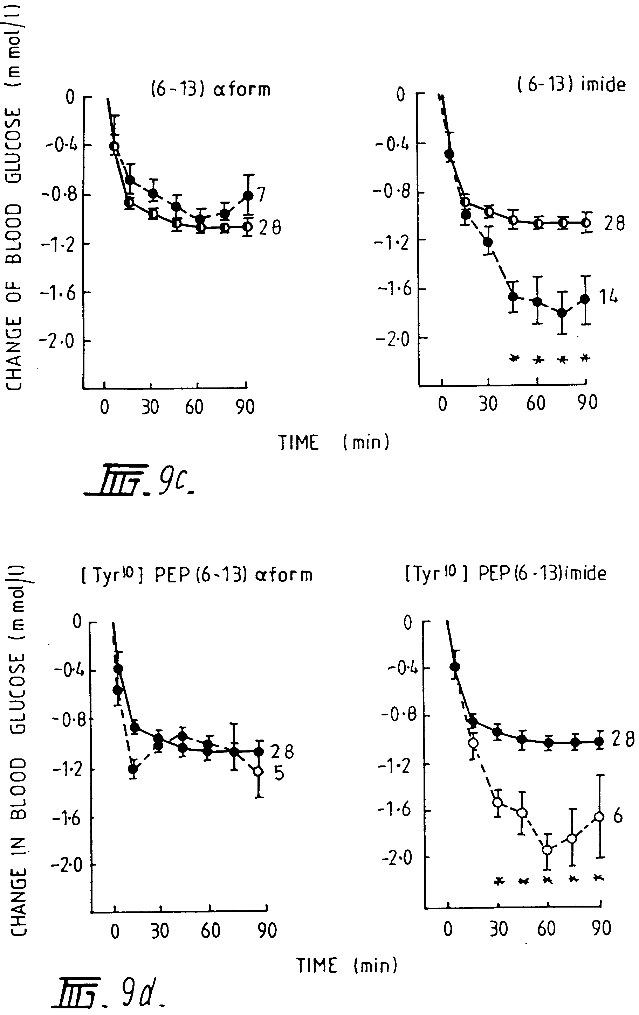

Similar results are illustrated graphically in Figure 9. The α form of Pep (6-13) was inactive for several variants tested (Ala¹¹, Gly¹⁰, Gly¹⁰Pro¹¹, Pro¹¹, Tyr¹⁰) whereas the β imide form was always active. Similarly Pep (6-11), Pep (6-12), Pep (6-13), Pep (6-15) were active only in the β imide form. However, for Pep (9-13) neither the α form nor the β imide form was active in this test.

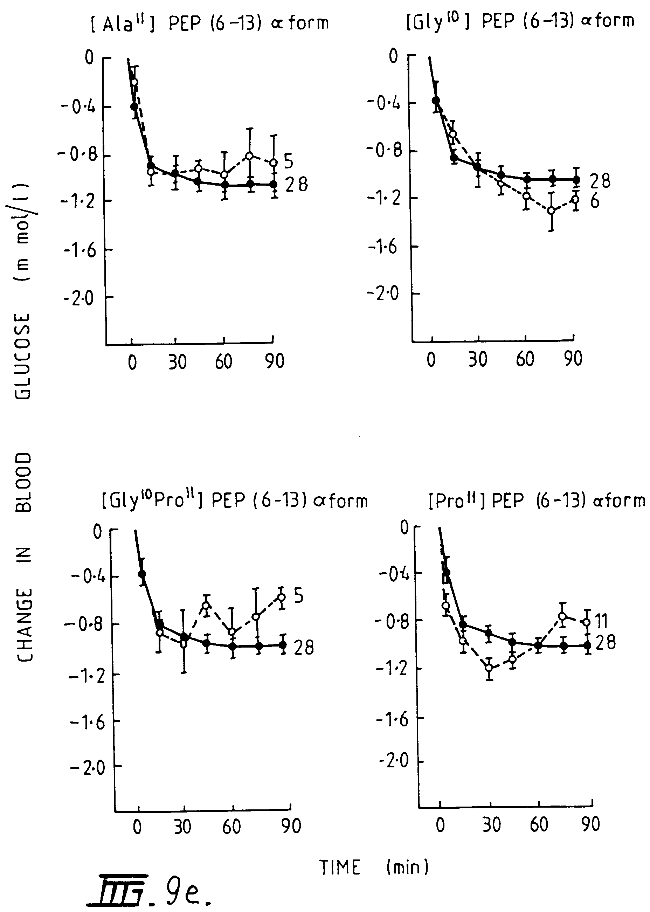

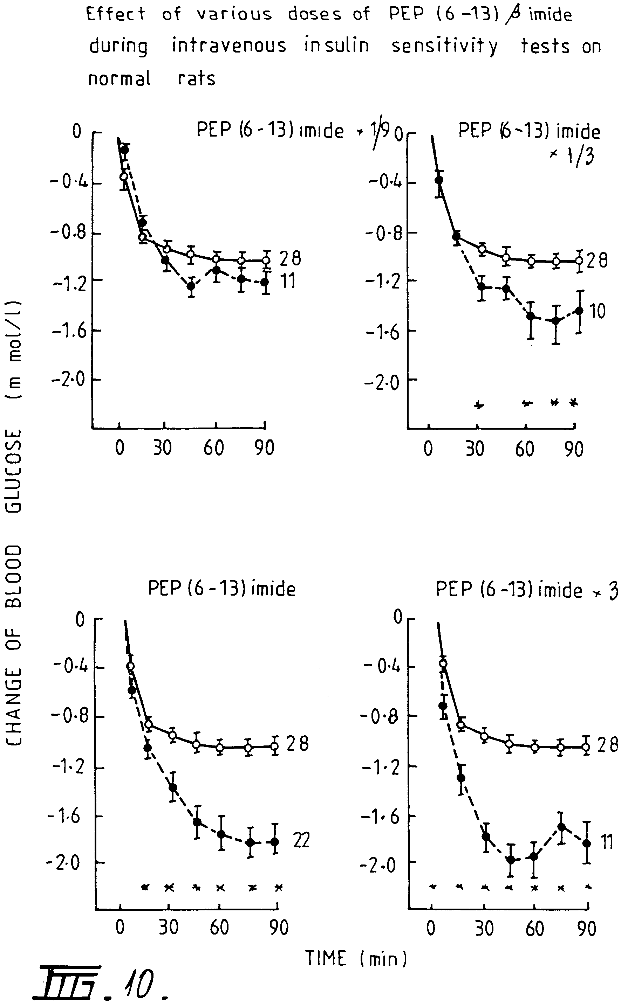

The β imide form of peptides according to the invention was also found to potentiate the action of insulin during intravenous insulin sensitivity tests carried out on rats trained to eat on a scheduled time basis. Rats were anaesthetised with pentobarbitone, and blood samples taken at various times after intravenous infusion of insulin. Control animals were untreated or were given insulin only; test animals received insulin plus the β imide form of Pep (6-13). The results are shown in Figure 10. The effect depended on the dose of peptide.

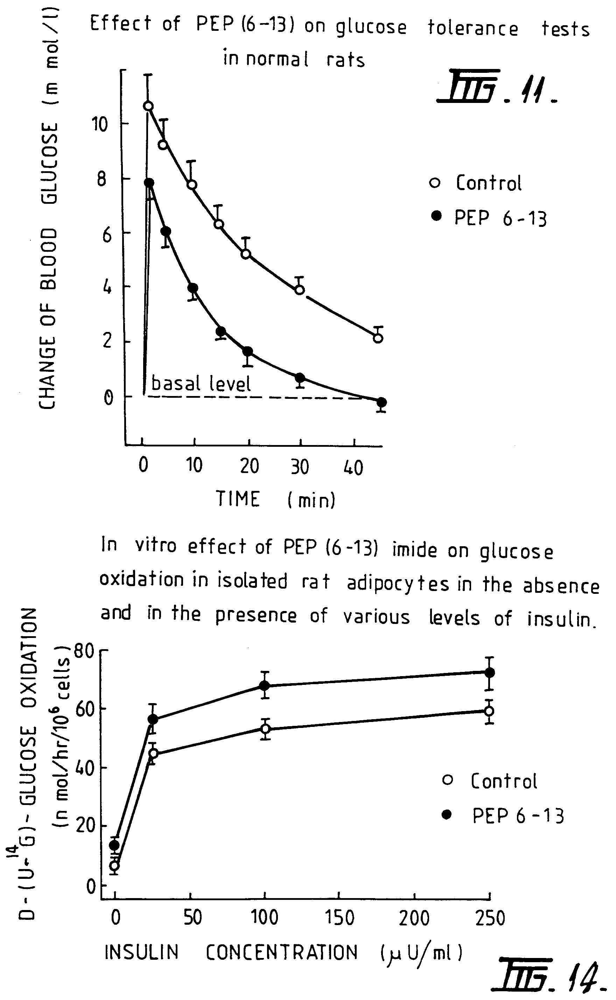

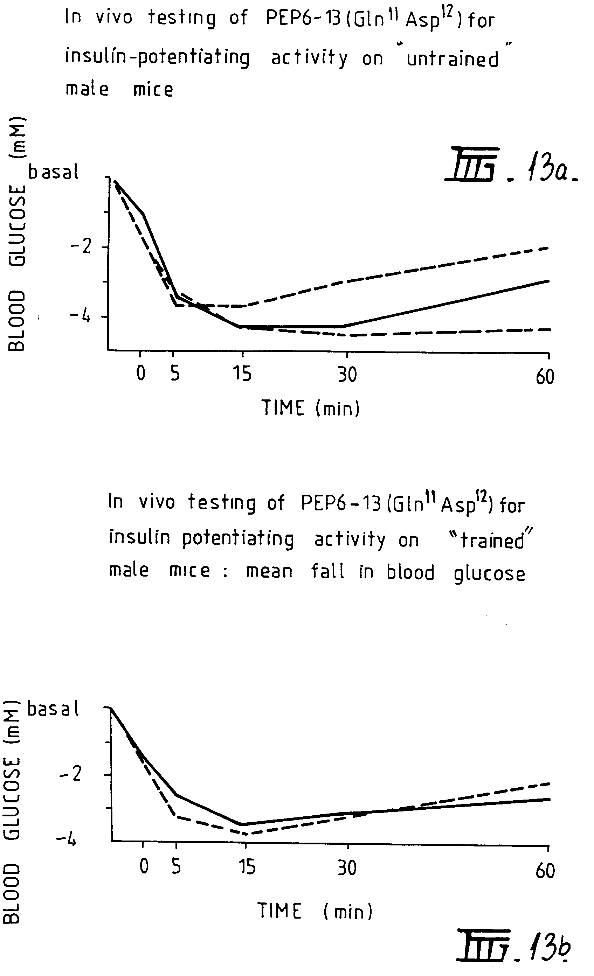

Figure 11 shows that Pep (6-13) β imide accelerates the return of blood glucose levels in rats to basal level in the glucose tolerance test. The dose of Pep (6-13) was 200 µg/Kg body weight. Similarly, various forms of the peptide according to the invention in its β imide form potentiate the hypoglycaemic effect of insulin on "untrained" male and female mice. The effect on trained male mice trained to feed on schedule as described above was less striking, but still significant in some cases. These results are illustrated in Figures 12 and 13.

As well as showing activity in the in vitro assays described above, peptides according to the invention showed hypoglycaemic activity in in vitro bioassays. Table 3 shows the effect of Pep (6-13) on glycogen deposition by isolated rat hemidiaphragms. There was no effect on the basal level in the absence of added insulin. At low or high insulin concentrations (10² and 10⁴ µU/ml) respectively, Pep (6-13) β imide significantly increased glycogen deposition. The α form and the ring opened, hydrolysed β imide form were both inactive.

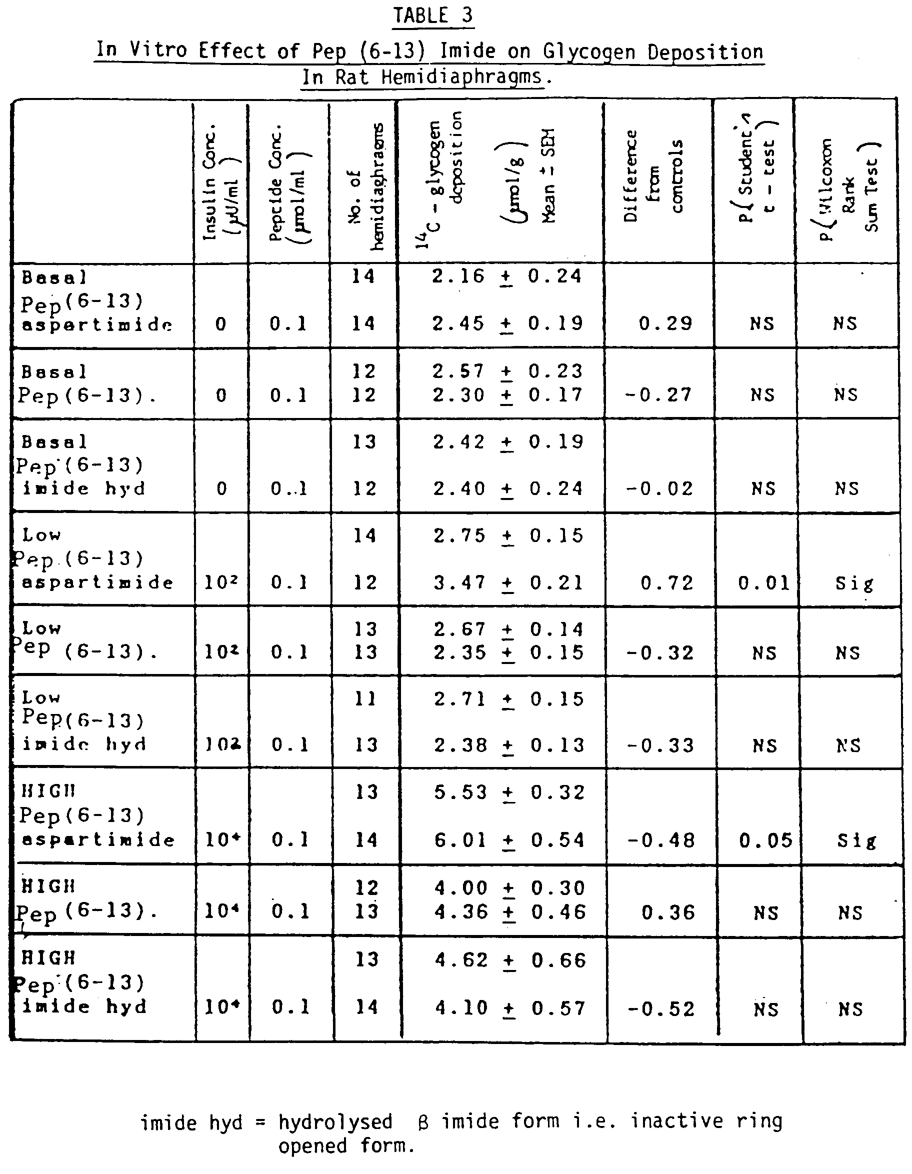

The β imide form of the peptide also stimulated metabolism of glucose by isolated rat adipocytes, both in the presence and in the absence of added insulin. This is shown in Figure 14. The degree of stimulation depended on insulin dose. The dose of peptide was 10 µg per 3 ml culture.

The effect of the β imide form peptides on glucose metabolism is further shown by their ability to stimulate glucose uptake and glycogen synthesis by cultured muscle cells and isolated rat hemidiaphragms.

The results are presented in Tables 4 and 5.

**TABLE 4**

| In Vitro Effect of β-Imido Linked L-Aspartyl-L-Asparaginyl Peptides On Glucose Uptake In Rat Hemidiaphragms. | | | | | | |
|---|---|---|---|---|---|---|
| Peptide | No. of Pairs | Peptide concentration (µmol/ml) | Glucose Uptake (µmol/g muscle/h) | | | *p* |
| | | | Control Mean ± SEM | + Peptide Mean ± SEM | Mean Difference | |
| 1-15 | 12 | 0.14 | 22.06 ± 1.39 | 30.39 ± 1.72 | 8.33 ± 1.50 | <0.001 |
| 6-13 | 15 | 0.16 | 35.34 ± 1.54 | 38.49 ± 1.53 | 3.15 ± 0.75 | <0.001 |
| 7-13 | 9 | 0.22 | 42.16 ± 1.88 | 46.79 ± 1.10 | 4.64 ± 1.63 | <0.25 |
| 8-13 | 7 | 0.20 | 38.70 ± 0.77 | 44.11 ± 0.69 | 5.41 ± 0.65 | <0.001 |
| 9-13 | 8 | 0.20 | 31.50 ± 1.01 | 32.16 ± 0.97 | 0.68 ± 0.80 | nsd |
| 10-13 | 9 | 0.20 | 33.86 ± 1.19 | 33.07 ± 1.01 | 0.80 ± 1.16 | nsd |

**TABLE 5**

| Results of Cultured Cell Assays for the Stimulation of Glucose Uptake by Synthetic β-Aspartimido Peptides (10µg/ml) | |
|---|---|
| Peptide | Rate of Increase from Basal (µmol glucose/hour) |
| P015 Ser-Arg-Leu-Phe-Asp-Asn-Ala | 1.5 |
| P051 Leu-Ser-Arg-Leu-Phe-Asp-Asn-Ala | 3.3 |
| P057 Leu-Ser-Arg-Leu-dPhe-Asp-Asn-Ala | 2.0 |
| P063 Leu-Ser-Arg-Leu-Phe-Asp-Asn-Ala-Gly | 1.9 |
| P086 Leu-Ser-Arg-Val-Phe-Asp-Asn-Ala | 4.9 |
| P168 Cys-Tyr-Leu-Ser-Arg-Leu-Phe-Asp-Asn-Ala | 4.0 |
| P202 Cys-Tyr-Leu-Ser-Arg-Leu-Phe-Asp-Asn-Ala | 1.9 |

| Results of Isolated Muscle Assays for the Stimulation of Glycogen Synthesis by Synthetic β-Aspartimido Peptides (100µg/ml) | |
|---|---|
| Peptide | Rate of Increase from Basal (µmol glycogen/g tissue/h) |
| P051 Leu-Ser-Arg-Leu-Phe-Asp-Asn-Ala | 0.74 |
| P076 Leu-Ser-Arg-Leu-Phe-Asp-Gln-Ala | 0.30 |
| P078 Leu-Ser-Arg-Leu-His-Asp-Asn-Ala | 0.11 |
| P087 Leu-Ser-Arg-Ile-Phe-Asp-Asn-Ala | 0.36 |
| P090 Leu-Ser-Lys-Leu-Phe-Asp-Asn-Ala | 0.16 |
| P197 Cys-Tyr-Leu-Ser-Arg-Leu-Phe-Asp-Asn-Asn | 0.17 |
| P202 Cys-Tyr-Leu-Ser-Arg-Leu-Phe-Asp-Asn-Ala | 0.44 |

Of the peptides tested, Pep (1-15), (6-13), and (8-13) showed a highly significant stimulation; Pep (7-13) was on the borderline of significance, while Pep (9-13) and Pep (10-13) showed no effect.

The results show that peptides of general formula I induce in a dose dependent manner a significant lowering of blood glucose levels at or below the level of 10µM/Kg body weight with the intravenous insulin tolerance test, and enhance in a dose dependent manner glucose utilisation via activation of glycogen synthase, incorporation of glucose into glycogen and oxidation of glucose via pyruvate to carbon dioxide. A dose of as little as 0.05 µM/Kg body weight was effective with some peptides.

### APPLICATIONS OF THE INVENTION

The β-imido-L-aspartyl-L-asparaginyl peptides of general formula I induce
(a) dose-dependent lowering of blood glucose and a potentiation of glycogen synthesis in vivo
(b) dose-dependent oxidation of glucose and the activation of glycogen synthase in vitro.

This suggests that the β-imido-L-aspartyl-L-asparaginyl peptide analogues are potent hypoglycaemic agents, and may be useful as therapeutic agents for the control of glucose metabolism, since normalisation of blood glucose levels in insulin-independent diabetes mellitus (Type II) mammals with glucose intolerance and insulin resistance is achieved by control of carbohydrate metabolism via glucose utilisation, and since glucose utilisation can be achieved via two pathways, namely oxidation of glucose or incorporation of glucose into glycogen.

Since potentiation of insulin action results in an decreased requirement for endogenous insulin, the use of the β-imido-L-aspartyl-L-asparaginyl peptide analogues may permit control of hyperinsulinaemia in insulin-independent Type II diabetes in humans or streptozotocin-treated animals.

(f) By analogy, the use of other synthetic peptide analogues containing the β-imido-L-aspartyl-L-asparaginyl moiety of general formula I is likely to exhibit either agonistic or antagonistic properties in vitro or in vivo related to glucose utilisation and so provide methods for control of hypoglycaemia or hyperglycaemia, depending on their respective agonistic or antagonistic properties in control of glucose metabolism.

It will be clearly understood that the invention in its general aspects is not limited to the specific details referred to hereinabove.

The following words used hereinabove are trade marks: BIOGEL, NUJOL.

## Claims

1. A compound of a general formula I: 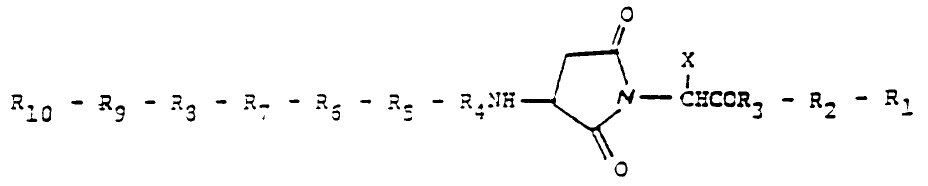 wherein X is hydrogen, -CH₂CONH₂ or -CH₂CH₂CONH₂;
each of R₁ and R₂ can be absent or is an L-α-amino acid, a δ-amino acid or an ε-amino acid;
R₃ is an L-α-amino acid, a δ-amino acid or an ε-amino acid;
R₄ is an L or D α-amino acid, a δ-amino acid or an ε-amino acid;
each of R₅ to R₇ is a bond, hydrogen or an L or D α-amino acid, a δ-amino acid or an ε-amino acid;
and each of R₈ to R₁₀ can be absent or is a hydrogen, or an L or D α-amino acid, a δ-amino acid or an ε-amino acid;
or a pharmaceutically acceptable salt thereof, with the proviso that the compound is not L-His-L-Ser-α-aminosuccinimido-Gly-L-Thr-L-Phe
or
L-Val-L-Tyr-L-Pro-α-aminosuccinimido-Gly-Ala.

2. A compound according to Claim 1 in which R₅ is a L or D α-amino acid, a δ-amino acid or an ε-amino acid.

3. A compound according to Claim 1 or Claim 2 in which each of R₁, R₂ and R₃ is selected from the group consisting of alanine, glycine and phenylalanine.

4. A compound according to any one of Claims 1 to 3, in which R₄ is a hydrophobic amino acid.

5. A compound according to Claim 4 in which R₄ is selected from the group consisting of L-phenylalanine, L-tyrosine, L-tryptophan and L-histidine.

6. A compound according to any one of the preceding claims, in which R₅ is a hydrophobic amino acid.

7. A compound according to Claim 6 in which R₅ is selected from the group consisting of L- or D- leucine, isoleucine and histidine.

8. A compound according to any one of the preceding claims, in which R₆ is a basic amino acid.

9. A compound according to Claim 8, in which R₆ is selected from the group consisting of L- or D-arginine, lysine, and histidine.

10. A compound according to any one of the preceding claims, in which R₇ is a substantially hydrophobic amino acid.

11. A compound according to Claim 10 in which R₇ is selected from the group consisting of L- or D- serine, leucine and isoleucine.

12. A compound according to any one of the preceding claims, in which R₈ is selected from the group consisting of L- or D- leucine, phenylalanine, proline or isoleucine, an ε-amino acid such as 6-aminohexanoic acid, and 4-aminocyclohexane-1-carboxylic acid.

13. A compound according to any one of the preceding claims, in which each of R₉ and R₁₀ is a substantially hydrophobic amino acid.

14. A compound according to Claim 13, in which each of R₉ and R₁₀ is selected from the group consisting of L- or D-leucine, phenylalanine, proline, isoleucine or tyrosine.

15. A compound according to any one of the preceding claims, in which one of R₈, R₉ or R₁₀ is coupled to R₁ via a bifunctional agent.

16. A compound according to Claim 15, in which the bifunctional agent is 6-aminohexanoic acid.

17. A compound according to any one of the preceding claims, in which one or more of the amino acids has side chain substituents.

18. A compound according to any one of the preceding claims, in which R₁, R₂, R₈, R₉, or R₁₀ is absent from the peptide, allowing coupling between C-terminal and N-terminal amino acids via a bifunctional agent such as 6-aminohexanoic acid.

19. A pharmacologically active analogue or derivative of a compound according to any one of the preceding claims, in which the aspartimide ring is replaced by a group selected from o-aminobenzoic acid, m-aminobenzoic acid, p-aminobenzoic acid, α-lactams and δ-lactams.

20. A pharmaceutical composition comprising a compound according to any of claims 1 to 19 and/or the compound L-His-L-Ser-α-aminosuccinimido-Gly-L-Thr-L-Phe and/or L-Val-L-Tyr-L-Pro-α-aminosuccinimido-Gly-Ala together with a pharmaceutical acceptable carrier, diluent or excipients.

21. A method of solid phase synthesis of a peptide of general formula I, in which the peptide is bound to a resin, comprising the steps of:
(a) protecting the α-amino functionality of added amino acids using t-BOC or f-MOC,
(b) treating the resin-bound protected α/straight chain L-aspartyl-L-asparaglnyl peptide with a base to produce the stable β-imido form of the peptide, and
(c) recovering the peptide.

22. A method according to Claim 21, in which the base is a dialkylamine or a trialkylamine.

23. A method according to Claim 22, in which the base is piperidine or triethylamine.

24. Use of a compound according to any of claims 1 to 19 and/or L-His-L-Ser-α-aminosuccinimido-Gly-L-Thr-L-Phe and/or L-Val-L-Tyr-L-Pro-α-aminosuccinimido-Gly-Ala for preparing a medicine for lowering the level of blood glucose in a mammal.

## Patentansprüche

1. Eine Verbindung einer allgemeinen Formel I: 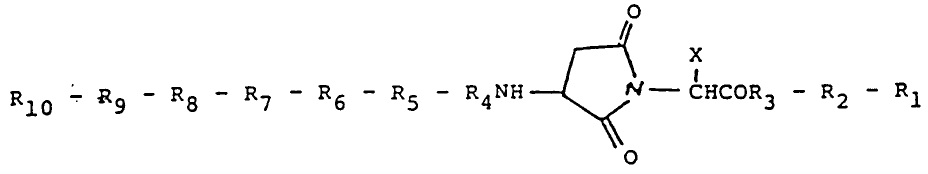 worin X Wasserstoff, -CH₂CONH₂ oder -CH₂CH₂CONH₂ darstellt;
R₁ und R₂ können jeweils abwesend sein oder stellen eine L-α-Aminosäure, eine δ-Aminosäure oder eine ε-Aminosäure dar;
R₃ stellt eine L-α-Aminosäure, eine δ-Aminosäure oder eine ε-Aminosäure dar;
R₄ stellt eine L- oder D-α-Aminosäure, eine δ-Aminosäure oder eine ε-Aminosäure dar;
R₅ bis R₇ stellen jeweils eine Bindung, Wasserstoff oder eine L- oder D-α-Aminosäure, eine δ-Aminosäure oder eine ε-Aminosäure dar;
und R₈ bis R₁₀ können jeweils abwesend sein oder stellen ein Wasserstoffatom oder eine L- oder D-α-Aminsäure, eine δ-Aminosäure oder eine ε-Aminosäure dar;
oder ein pharmazeutisch zulässiges Salz davon, mit der Maßgabe, daß die Verbindung nicht L-His-L-Ser-α-Aminosuccinimido-Gly-L-Thr-L-Phe oder L-Val-L-Tyr-L-Pro-α-Aminosuccinimido-Gly-Ala darstellt.

2. Eine Verbindung nach Anspruch 1, in der R₅ eine L- oder D-α-Aminosäure, eine δ-Aminsäure oder eine ε-Aminosäure darstellt.

3. Eine Verbindung nach Anspruch 1 oder 2, in der R₁, R₂ und R₃ jeweils ausgewählt wird aus der Gruppe bestehend aus Alanin, Glycin und Phenylalanin.

4. Eine Verbindung nach einem der Ansprüche 1 bis 3, in der R₄ eine hydrophobe Aminosäure darstellt.

5. Eine Verbindung nach Anspruch 4, in der R₄ ausgewählt wird aus der Gruppe bestehend aus L-Phenylalanin, L-Tyrosin, L-Tryptophan und L-Histidin.

6. Eine Verbindung nach einem der vorhergehenden Ansprüche, in der R₅ eine hydrophobe Aminosäure darstellt.

7. Eine Verbindung nach Anspruch 6, in der R₅ ausgewählt wird aus der Gruppe bestehend aus L- oder D-Leucin, Isoleucin und Histidin.

8. Eine Verbindung nach einem der vorhergehenden Ansprüche, in der R₆ eine basische Aminosäure darstellt.

9. Eine Verbindung nach Anspruch 8, in der R₆ ausgewählt wird aus der Gruppe bestehend aus L- oder D-Arginin, Lysin und Histidin.

10. Eine Verbindung nach einem der vorhergehenden Ansprüche, in der R₇ eine im wesentlichen hydrophobe Aminosäure darstellt.

11. Eine Verbindung nach Anspruch 10, in der R₇ ausgewählt wird aus der Gruppe bestehend aus L- oder D-Serin, Leucin und Iso-leucin.

12. Eine Verbindung nach einem der vorhergehenden Ansprüche, in der R₈ ausgewählt wird aus der Gruppe bestehend aus L- oder D-Leucin, Phenylalanin, Prolin oder Isoleucin, eine ε-Aminosäure, wie 6-Aminohexansäure und 4-Aminocyclohexan-1-Karbonsäure.

13. Eine Verbindung nach einem der vorhergehenden Ansprüche, in der R₉ und R₁₀ jeweils eine im wesentlichen hydrophobe Aminosäure darstellt.

14. Eine Verbindung nach Anspruch 13, in der R₉ und R₁₀ jeweils ausgewählt werden aus der Gruppe bestehend aus L- oder D-Leucin, Phenylalanin, Prolin, Isoleucin oder Tyrosin.

15. Eine Verbindung nach einem der vorhergehenden Ansprüche, in der eine von R₈, R₉ oder R₁₀ an R₁ über ein bifunktionelles Agenz gekuppelt ist.

16. Eine Verbindung nach Anspruch 15, in der das bifunktionelle Agenz 6-Aminohexansäure ist.

17. Eine Verbindung nach einem der vorhergehenden Ansprüche, in der eine oder mehrere der Aminosäuren Seitenkettensubstituenten trägt.

18. Eine Verbindung nach einem der vorhergehenden Ansprüche, in der R₁, R₂, R₈, R₉ oder R₁₀ von dem Peptid abwesend ist, wobei dies eine Kupplung zwischen den C-terminalen und N-terminalen Aminosäuren über ein bifunktionelles Agenz wie 6-Aminohexansäure ermöglicht.

19. Pharmakologisch aktives Analogon oder Derivat einer Verbindung nach einem der vorhergehenden Ansprüche, in dem der Aspartimidring ersetzt ist durch eine Gruppe ausgewählt aus o-Aminobenzoesäure, m-Aminobenzoesäure, p-Aminobenzoesäure, α-Lactamen und δ-Lactamen.

20. Eine pharmazeutische Zusammensetzung umfassend eine Verbindung nach einem der Ansprüche 1 bis 19 und/oder die Verbindung L-His-L-Ser-α-Aminosuccinimido-Gly-L-Thr-L-Phe und/oder L-Val-L-Tyr-L-Pro-α-Aminosuccinimido-Gly-Ala zusammen mit einem pharmazeutisch zulässigen Träger, Verdünnungsmittel oder Hilfsstoffen.

21. Ein Verfahren für die Festphasensynthese eines Peptids der allgemeinen Formel I, in dem das Peptid an ein Harz gebunden ist, umfassend die Schritte:
a) Schützen der α-Aminofunktion der zugesetzten Aminosäuren mit Hilfe von t-BOC oder f-MOC,
b) Behandeln des harzgebundenen, geschützten, geradkettigen L-Aspartyl-L-Asparaginyl-α-Peptids mit einer Base, um die stabile β-Imidoform des Peptids auszubilden, und
c) gewinnen des Peptids.

22. Ein Verfahren nach Anspruch 21, in dem die Base ein Dialkylamin oder Trialkylamin darstellt.

23. Ein Verfahren gemäß Anspruch 22, in dem die Base Piperidin oder Triethylamin darstellt.

24. Verwendung einer Verbindung nach einem der Ansprüche 1 bis 19 und/oder L-His-L-Ser-α-Aminosuccinimido-Gly-L-Thr-L-Phe und/oder L-Val-L-Tyr-L-Pro-α-Aminosuccinimido-Gly-Ala zum Herstellen eines Medikaments zum Erniedrigen des Spiegels an Blutglukose in einem Säuger.

## Revendications

1. Composé de formule générale I : 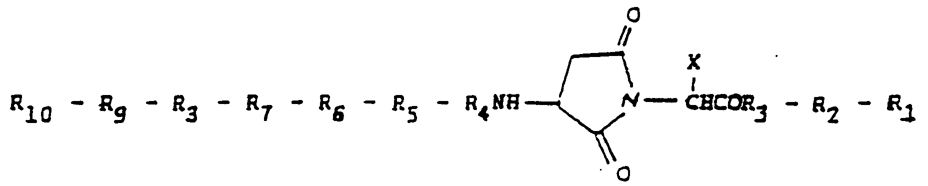 dans laquelle X est un hydrogène, -CH₂CONH₂ ou -CH₂CH₂CONH₂ ;
chacun de R₁ et R₂ peut être absent ou est un acide L-α-aminé, un acide δ-aminé ou un acide ε-aminé ;
R₃ est un acide L-α-aminé, un acide δ-aminé ou un acide ε-aminé ;
R₄ est un acide L ou D-α-aminé, un acide δ-aminé ou un acide ε-aminé ;
chacun de R₅ à R₇ est une liaison, un hydrogène ou un acide L ou D-α-aminé, un acide δ-aminé ou un acide ε-aminé ;
et chacun de R₈ à R₁₀ peut être absent ou est un hydrogène, ou un acide L ou D-α-aminé, un acide δ-aminé ou un acide ε-aminé ;
ou un sel pharmaceutiquement acceptable de celui-ci, avec la condition que le composé ne soit pas le composé L-His-L-Ser-α-aminosuccinimido-Gly-L-Thr-L-Phe ni L-Val-L-Tyr-L-Pro-α-aminosuccinimid-Gly-Ala.

2. Composé selon la revendication 1, dans lequel R₅ est un acide L ou D-α-aminé, un acide δ-aminé ou un acide ε-aminé.

3. Composé selon la revendication 1 ou la revendication 2, dans lequel chacun de R₁, R₂ et R₃ est choisi dans le groupe constitué de l'alanine, de la glycine et de la phénylalanine.

4. Composé selon l'une quelconque des revendications 1 à 3, dans lequel R₄ est un acide aminé hydrophobe.

5. Composé selon la revendication 4, dans lequel R₄ est choisi dans le groupe constitué de la L-phényl-alanine, de la L-tyrosine, du L-tryptophane et de la L-histidine.

6. Composé selon l'une quelconque des revendications précédentes, dans lequel R₅ est un acide aminé hydrophobe.

7. Composé selon la revendication 6, dans lequel R₅ est choisi dans le groupe constitué de la L- ou D-leucine, de l'isoleucine et de l'histidine.

8. Composé selon l'une quelconque des revendications précédentes, dans lequel R₆ est un acide aminé basique.

9. Composé selon la revendication 8, dans lequel R₆ est choisi dans le groupe constitué de la L- ou D-arginine, de la lysine et de l'histidine.

10. Composé selon l'une quelconque des revendications précédentes, dans lequel R₇ est un acide aminé substantiellement hydrophobe.

11. Composé selon la revendication 10, dans lequel R₇ est choisi dans le groupe constitué de la L- ou D-sérine, de la leucine et de l'isoleucine.

12. Composé selon l'une quelconque des revendications précédentes, dans lequel R₈ est choisi dans le groupe constitué de la L- ou D-leucine, de la phénylalanine, de la proline ou de l'isoleucine, d'un acide ε-aminé tel que l'acide 6-aminohexanoïque, et de l'acide 4-aminocyclohexane-1-carboxylique.

13. Composé selon l'une quelconque des revendications précédentes, dans lequel chacun de R₉ et R₁₀ est un acide aminé substantiellement hydrophobe.

14. Composé selon la revendication 13, dans lequel chacun de R₉ et R₁₀ est choisi dans le groupe constitué de la L- ou D-leucine, de la phénylalanine, de la proline, de l'isoleucine ou de la tyrosine.

15. Composé selon l'une quelconque des revendications précédentes, dans lequel l'un de R₈, R₉ ou R₁₀ est couplé à R₁ par l'intermédiaire d'un agent bifonctionnel.

16. Composé selon la revendication 15, dans lequel l'agent bifonctionnel est l'acide 6-aminohexanoïque.

17. Composé selon l'une quelconque des revendications précédentes, dans lequel un ou plusieurs des acides aminés a des substituants de chaîne latérale.

18. Composé selon l'une quelconque des revendications précédentes, dans lequel R₁, R₂, R₈, R₉ ou R₁₀ est absent du peptide, permettant le couplage entre les acides C-terminaux et N-terminaux par l'intermédiaire d'un agent bifonctionnel tel que l'acide 6-aminohexanoïque.

19. Analogue ou dérivé pharmacologiquement actif d'un composé selon l'une quelconque des revendications précédentes, dans lequel le cycle aspartimide est remplacé par un groupe choisi parmi l'acide o-aminobenzoïque, l'acide m-aminobenzoïque, l'acide p-aminobenzoïque, les α-lactames et les δ-lactames.

20. Composition pharmaceutique comprenant un composé selon l'une quelconque des revendications 1 à 19 et/ou le composé L-His-L-Ser-α-aminosuccinimido-Gly-L-Thr-L-Phe et/ou L-Val-L-Tyr-L-Pro-α-aminosuccinimido-Gly-Ala avec un véhicule, un diluant ou des excipients pharmaceutiquement acceptables.

21. Procédé de synthèse en phase solide d'un peptide de formule générale I, dans lequel le peptide est lié à une résine, comprenant les étapes consistant :
(a) à protéger la fonctionnalité α-amino des acides aminés ajoutés en utilisant t-BOC ou f-MOC,
(b) à traiter le peptide L-aspartyl-L-asparaginyl à chaîne linéaire/α protégé, lié à une résine, avec une base pour produire la forme β-imido stable du peptide, et
(c) à récupérer le peptide.

22. Procédé selon la revendication 21, dans lequel la base est une dialkylamine ou une trialkylamine.

23. Procédé selon la revendication 22, dans lequel la base est la pipéridine ou la triéthylamine.

24. Utilisation d'un composé selon l'une quelconque des revendications 1 à 19 et/ou de L-His-L-Ser-α-aminosuccinimido-Gly-L-Thr-L-Phe et/ou de L-Val-L-Tyr-L-Pro-α-aminosuccinimido-Gly-Ala pour préparer un médicament pour abaisser la glycémie chez un mammifère.
